# EUROPEAN PATENT APPLICATION

(11) **EP 4 372 077 A1**
(43) Date of publication of application: **22.05.2024**
(21) Application number: 22842098.0
(22) Date of filing: 12.07.2022
(51) Int. Cl.: C12N 1/20, A61K 35/747, A61P 1/00, A61P 43/00, C12R 1/23, C12R 1/245, C12R 1/25

(54) **AUTOPHAGY-ACTIVATING COMPOSITION**

(30) Priority: 12.07.2021 JP 2021114935
(71) Applicant: National University Corporation Tokyo Medical and Dental University, Tokyo 113-8510 (JP); MEIJI CO., LTD, Chuo-ku Tokyo 104-8306 (JP)
(72) Inventor: SHIMIZU Shigeomi, Tokyo 113-8510 (JP); WATANABE Yumiko, Hachioji-shi, Tokyo 192-0919 (JP); SASHIHARA Toshihiro, Hachioji-shi, Tokyo 192-0919 (JP)
(74) Representative: TBK
(86) International application number: PCT/JP2022/027341
(87) International publication number: WO 2023/286754

(57) **Abstract**

A composition comprising an ingredient that is effective for inducing autophagy in intestinal epithelial cells and intestinal epithelial stem cells and can be ingested at ease on a daily basis is provided. A composition for inducing autophagy, the composition comprising bacterial cells of a lactic acid bacterium belonging to the family *Lactobacillaceae* is provided. The lactic acid bacterium is preferably a lactic acid bacterium belonging to any of the genera *Lactiplantibacillus*, *Lactobacillus*, *Lacticaseibacillus*, *Companilactobacillus*, and *Limosilactobacillus.*

## Description

### Technical Field

The present invention relates to a composition for inducing autophagy, which contains a specific lactic acid bacterium as an active ingredient.

### Background Art

Autophagy is a system that takes up substrates to be degraded by autophagosomes, which have a double membrane structure, and degrades their contents by fusion of them with lysosomes. Autophagy is a mechanism for degrading intracellular degenerated proteins and damaged organelles, and is important for maintaining homeostasis in living organisms.

Paneth cells present in the crypt of the small intestine release antibacterial peptides to prevent the entry of bacteria into the body, but inhibition of autophagy of these cells is known to allow intestinal bacteria to enter into the body and cause a strong inflammatory response (Non-patent document 1). Autophagy is thought to be important for the maintenance of Paneth cell function, since Paneth cell abnormalities have been observed in mice in which the intestinal epithelial cell-specific Atg5 and Atg7 were knocked out, and the number of mature Paneth cells is reduced in Crohn's disease patients (Non-patent document 2). Furthermore, intestinal epithelial stem cells (ISCs), which exist next to Paneth cells in the crypt, have high self-renewal ability and differentiation ability into epithelial cells, and it has been reported that autophagy of these cells is essential for maintenance of intestinal epithelial cells and regeneration of the cells after intestinal epithelial injury through regulation of production of reactive oxygen species (ROS), and so forth (Non-patent document 3). It has also been reported that inhibition of autophagy in the intestinal epithelium causes abnormalities in the function of intestinal stem cells and mucin secretion from goblet cells, resulting in disruption of the intestinal barrier (Non-patent document 4).

By the way, functions to ameliorate diseases or undesirable conditions have been reported for certain types of lactic acid bacteria. For example, it has been reported that mice with high fat diet-induced obesity treated with 10⁹ cfu of heat-sterilized lactic acid bacteria for 12 weeks showed significant decreases in blood glucose levels in response to insulin and significant decreases in expression of interleukin-1β mRNA in adipose tissue and fatty acid concentration in serum (Non-patent document 5). It has also been reported that optimization of culture conditions is important for the production of lactic acid bacteria with anti-inflammatory activity on the basis of activity evaluation in mouse immune cells and obese type 2 diabetic mice (Non-patent document 6 and Patent document 1). Furthermore, it has been reported that when dendritic cells (DCs) purified from mesenteric lymph nodes (MLNs) or Peyer's patches (PPs) were co-cultured with lactic acid bacteria, IL-10 mRNA expression and protein production were increased in both types of DCs; when antigen-specific responses were allowed in T cells co-cultured with DCs, the addition of lactic acid bacteria enhanced IL-10 production in the T cells; and mice orally administered with 4 mg/day of heat-sterilized lactic acid bacteria for 6 days showed increased IL-10 gene expression in MLN DCs, and increased IL-10 gene expression in PP DCs after 12 hours from the oral administration (Non-patent document 7). Furthermore, it has been reported that daily consumption of yogurt containing at least 5 × 10⁹ heat-treated cells of lactic acid bacteria for 12 weeks by overweight (BMI of 25 to 30 kg/m² or higher) adults aged 20 to 64 years significantly reduced FBG (fasting blood glucose) levels in a subgroup with high FBG levels, and also suppressed fat accumulation, deterioration of glycemic control, and chronic inflammation (Non-patent documents 8 and 9). Patent document 2 describes a composition for suppressing accumulation of visceral fat, which contains a culture of *Lactobacillus plantarum* OLL2712 strain (accession number, FERM BP-11262) or a processed product thereof. Patent document 3 describes a composition for improving human glucose metabolism, which contains lactic acid bacteria belonging to the genus *Lactobacillus* and has an activity to reduce the amount of at least one inflammatory cytokine selected from MCP-1 and IL-6 in human blood. Furthermore, Patent document 4 describes an agent for improving mitochondrial function, which contains lactic acid bacteria belonging to *Lactobacillus farciminis* or *Pediococcus acidilactici*, a culture of the lactic acid bacteria, or a culture supernatant of the lactic acid bacteria. This reference also describes that this agent can be used for improving metabolic function, extending life span, preventing aging, improving decreased motor ability, inhibiting muscle weakness, restoring nervous system abnormalities, or improving cognitive function.

In relation to autophagy and its effects on the intestinal tract, with respect to certain lactic acid bacteria, there have been reported an ability to suppress disruption of the intestinal barrier function (Non-patent documents 10 to 12), ability to suppress gluten-specific enteropathy (Non-patent document 13), induction of autophagy-induced cell death by *Lactobacillus acidophilus* NCFM surface layer proteins in in vitro studies (Non-patent document 14), and promotion of autophagy by *Lactobacillus rhamnosus* GG in *Salmonella*-infected intestinal epithelial cells via expression of membrane ATG16L1 protein (Non-patent document 15).

### Prior Art References

### Patent documents

Patent document 1: WO2018/038004
Patent document 2: WO2012/014971 (Japanese Patent No. 5076029)
Patent document 3: Japanese Patent Unexamined Publication (Kokai) No. 2021-031408
Patent document 4: WO2020/067368

### Non-patent documents

Non-patent document 1: Burger, E et al., Cell Host Microbe, 2018; 23(2):177-190, e4. doi: 10.1016/j.chom.2018.01.001
Non-patent document 2: Yano, Tamaki, Chemistry and Biology, 2015;53(7):468-472, https://doi.org/10.1271/kagakutoseibutsu.53.468
Non-patent document 3: Asano, J et al., Cell Rep., 2017;20(5):1050-1060, doi: 10.1016/j.celrep.2017.07.019
Non-patent document 4: Woldarska et al., NLRP6 inflammasome orchestrates the colonic host-microbial interface by regulating goblet cell mucus secretion, Cell, 2014,. 156: 5, pages 1045-1059
Non-patent document 5: Sakai, T et al., Journal of Nutritional Science, 2013; 59(2). https://doi.org/10.3177/jnsv.59.144
Non-patent document 6: Toshimitsu, T et al., Applied and Environmental Microbiology, 2017; 83(7):e03001-16, doi: 10.1128/AEM.03001-16
Non-patent document 7: Takano, T et al., Biosci Microbiota Food Health, 2020; 39(2): 39-44, doi: 10.12938/bmfh.19-019
Non-patent document 8: Toshimitsu, T et al., Nutrients, 2020; 12(2):374, doi: 10.3390/nul2020374
Non-patent document 9: Toshimitsu, T et al., Current Developments in Nutrition, 2021;5(2), https://doi.org/10.1093/cdn/nzab006
Non-patent document 10: Yanjun Cui et al., Oncotagert, 2017; 8(44): 77489-77499, doi.org/10.18632/oncotarget.20536
Non-patent document 11: Miyauchi, E et al., J. Dairy Sci., 2009; 92(6):2400-2408, doi.org/103168/jds.2008-1698
Non-patent document 12: Pradeep K Shukla et al., FASEB J., 2018; 32(11):6274-6292, doi.org/10.1096/fj.201800351R
Non-patent document 13: Ogita, T et al., Immunobiology, 2015; 220(6):701-710, doi.org/10.1016/j.imbio.2015.01.004
Non-patent document 14: Huifang Wang et al., Food Funct., 2019; 10:4102-4112, doi.org/10.1039/C9F000109C
Non-patent document 15: LW. T. Lai et al., Beneficial Microbes, 2019; 10(8):913-922, doi.org/10.3920/BM2019.0046

### Summary of the Invention

### Object to be achieved by the invention

As described above, autophagy is important in the secretion of mucus from intestinal goblet cells, maintenance of Paneth cell function, maintenance of intestinal epithelial stem cells, regeneration thereof after intestinal epithelial injury, and so forth. Therefore, if there is an ingredient that is effective for inducing autophagy in intestinal epithelial cells and intestinal epithelial stem cells and can be ingested at ease on a daily basis, it can be expected to be useful for maintenance of health and prevention of disease.

### Means for achieving the object

The present invention provides the followings.
[1] A composition for inducing autophagy, the composition comprising contains bacterial cells of a lactic acid bacterium belonging to the family *Lactobacillaceae.*
[2] The composition according to 1, wherein the lactic acid bacterium is a lactic acid bacterium belonging to any of the genera *Lactiplantibacillus*, *Lactobacillus*, *Lacticaseibacillus*, *Companilactobacillus*, and *Limosilactobacillus.*
[3] The composition according to 1 or 2, wherein the lactic acid bacterium is a lactic acid bacterium belonging to any of *Lactiplantibacillus plantarum, Lactobacillus acidophilus*, *Lactobacillus amylovorus*, *Lacticaseibacillus casei*, *Lacticaseibacillus paracasei*, *Companilactobacillus farciminis*, and *Limosilactobacillus mucosae.*
[4] The composition according to any one of 1 to 3, which is for inducing autophagy in the intestine.
[5] The composition according to any one of 1 to 4, which comprises 1 × 10⁹ or more of bacterial cells of the lactic acid bacterium per one unit.
[6] The composition according to any one of 1 to 5, which comprises 2 × 10¹¹ or more of bacterial cells of the lactic acid bacterium per one unit.
[7] The composition according to any one of 1 to 6, wherein the bacterial cells of the lactic acid bacterium are dead bacterial cells.
[8] The composition according to 7, wherein the dead bacterial cells are heat-treated dead bacterial cells.
[9] A composition comprising bacterial cells of a lactic acid bacterium belonging to the family *Lactobacillaceae* for use in a method for inducing autophagy; use of bacterial cells of a lactic acid bacterium belonging to the family *Lactobacillaceae* in manufacture of a composition for inducing autophagy; and a method for inducing autophagy, the method comprising administering a composition comprising bacterial cells of a lactic acid bacterium belonging to the family *Lactobacillaceae* to a subject (the method includes therapeutic and non-therapeutic methods, the same shall apply in the following descriptions).
[10] The composition, use, or method according to 9, wherein the lactic acid bacterium is a lactic acid bacterium belonging to any of the genera *Lactiplantibacillus*, *Lactobacillus*, *Lacticaseibacillus*, *Companilactobacillus*, and *Limosilactobacillus.*
[11] The composition, use, or method according to 9 or 10, wherein the lactic acid bacterium is a lactic acid bacterium belonging to any of *Lactiplantibacillus plantarum*, *Lactobacillus acidophilus*, *Lactobacillus amylovorus*, *Lacticaseibacillus casei*, *Lacticaseibacillus paracasei*, *Companilactobacillus farciminis*, and *Limosilactobacillus mucosae.*
[12] The composition, use, or method according to any one of 9 to 11, wherein the induction of autophagy is induction of autophagy in the intestine.
[13] The composition, use, or method according to any one of 9 to 12, wherein the composition comprises 1 × 10⁹ or more of bacterial cells of a lactic acid bacterium per one unit.
[14] The composition, use, or method according to any one of 9 to 13, wherein the composition comprises 2 × 10¹¹ or more of bacterial cells of a lactic acid bacterium per one unit.
[15] The composition, use, or method according to any one of 9 to 14, wherein the bacterial cells of a lactic acid bacterium are dead bacterial cells.
[16] The composition, use, or method according to 15, wherein the dead bacterial cells are heat-treated dead bacterial cells.

### Effects of the Invention

Autophagy is activated by the composition of the present invention.

According to the present invention, autophagy can be activated by using lactic acid bacteria cells, which have been used in a food from very early years. The active ingredient of the composition of the present invention is not cytotoxic and does not induce cell death.

### Brief Description of the Drawings

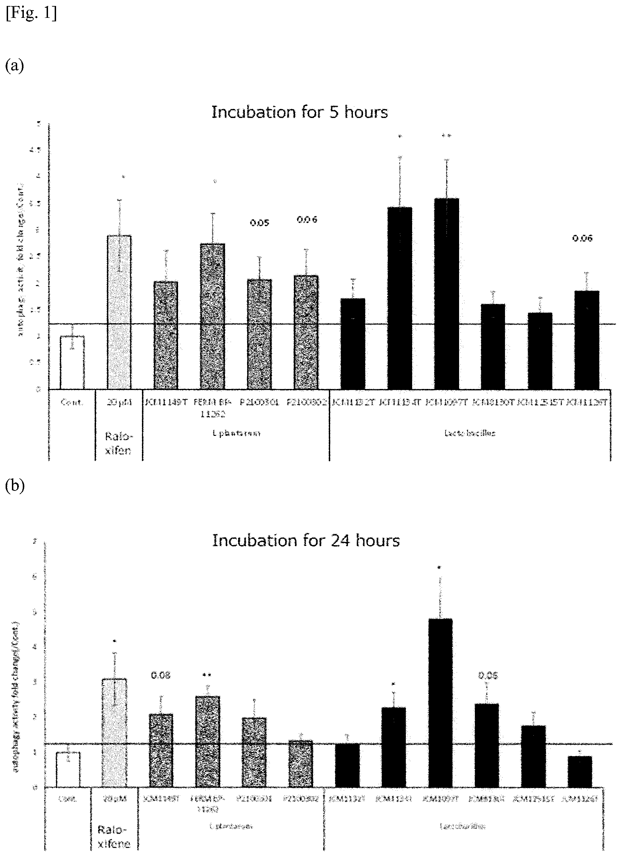

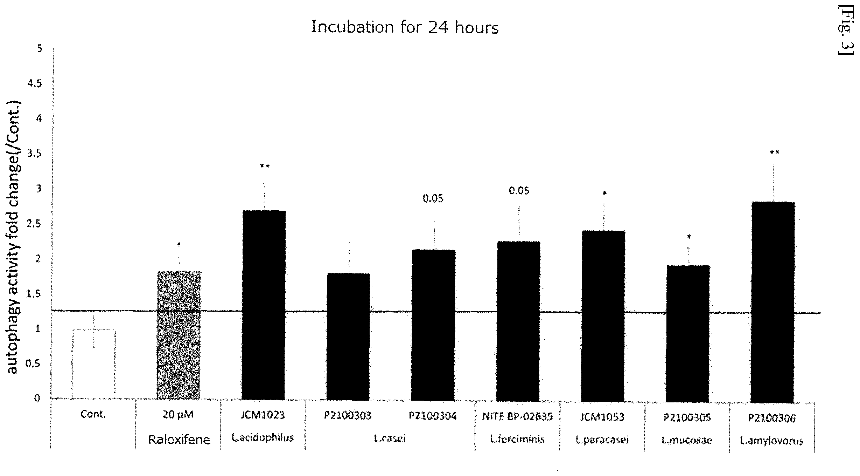

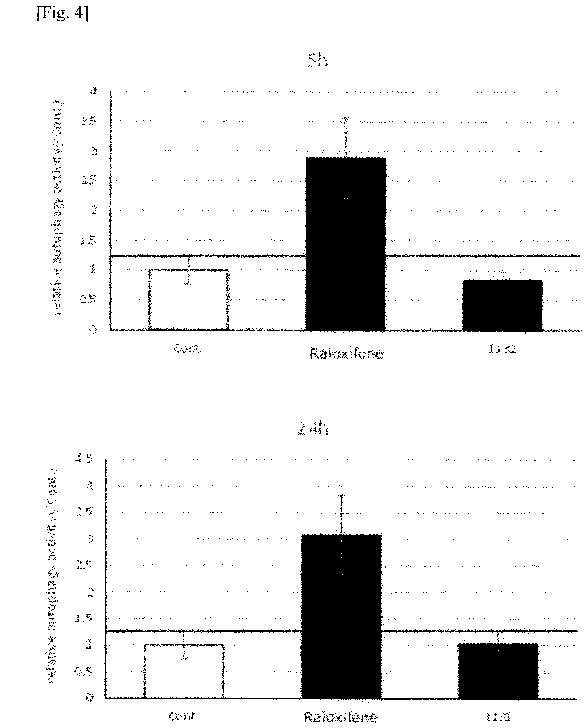

[Fig. 1] The results of autophagy activity measurement. The results are represented as mean ± SE (n=8 to 10/group). For each well, 5 fields of view were taken, and values obtained by dividing luminance (integrated) of luminescence of DALGreen by the number of cells for 10 fields of view in total for 2 wells of each sample were used as the luminescence intensities and compared with the value of Cont. (no stimulation), of which activity was taken as 1 (the same shall apply in Figs. 3 and 4). * indicates p<0.05, and ** indicates p<0.01 vs Cont. The numbers indicated over the columns indicate p-values. (a) Incubation for 5 hours, and (b) incubation for 24 hours.

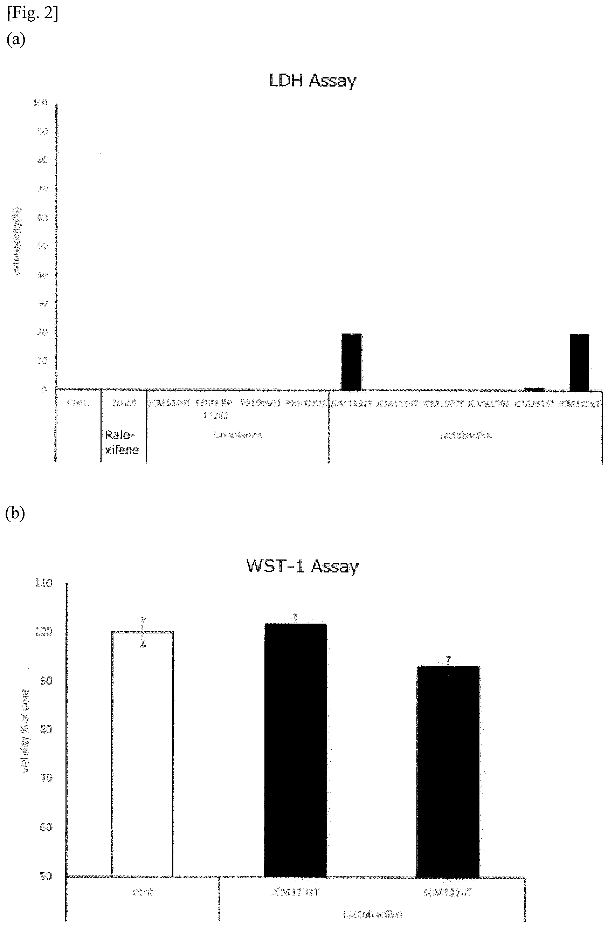

[Fig. 2] The results of cytotoxicity evaluation. (a) The results of LDH assay. The results are represented as mean ± SE (n=3/group). The results were calculated by using values obtained by subtracting absorbance of the blank from absorbance of each group (measured values) according to the following equation: {(Measured value for sample) - (Measured value for Cont.)}/{(Measured value for well in which cells were killed with lysis buffer) - (Measured value for Cont.)} × 100 (%). When the result was calculated to be a negative value, value of 0 was used instead. (b) The results of WST-1 assay. The results are represented as mean ± SE (n=3/group). Values obtained by subtracting absorbance of the blank from absorbance of each group (measured values) were compared with that of Cont. group.
[Fig. 3] The results of the autophagy activity measurement. The results are represented as mean ± SE (n=7-10/group). * indicates p<0.05, and ** indicates p<0.01 vs Cont. The numbers indicated over the columns indicate p-values.
[Fig. 4] The results of the autophagy activity assay. The results are represented as mean ± SE (n=9 or10/group). The abbreviation ralo means raloxifene; and 1131 means *Streptococcus thermophilus* 1131 strain.

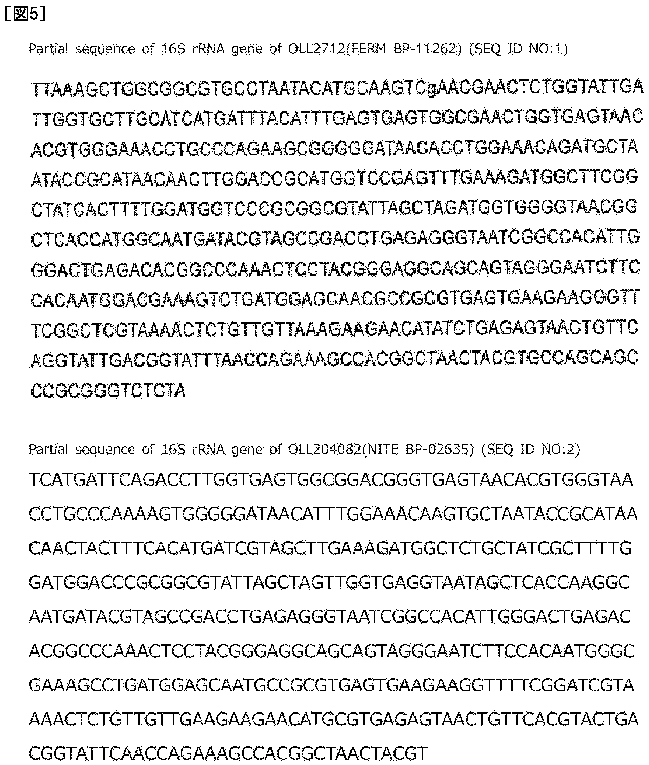

[Fig. 5] Partial sequence of 16S rRNA gene of OLL2712 (FERM BP-11262) (SEQ ID NO: 1) and partial sequence of 16S rRNA gene of OLL204082 (NITE BP-02635) (SEQ ID NO: 2).

### Modes for Carrying out the Invention

Hereafter, the present invention will be explained in detail.

The present invention relates to a composition comprising a lactic acid bacterium. More precisely, the present invention relates to a composition for inducing autophagy, which comprises a lactic acid bacterium as an active ingredient.

### [Active ingredient]

The composition of the present invention comprises a lactic acid bacterium belonging to the phylum *Firmicutes*, class *Bacilli*, order *Lactobacillales*, family *Lactobacillaceae.* According to the study of the inventors of the present invention, the desired function was not found in *Streptococcus thermophilus* of the family *Streptococcuaceae* of the same order under the conditions used for the experiments.

For the present invention, lactic acid bacteria are explained according to the classification based on the reclassification by Zheng J, Wittouck S, Salvetti E, Franz CMAP, Harris HMB, Mattarelli P, O'Toole PW, Pot B, Vandamme P, Walter J, Watanabe K, Wuyts S, Felis GE, Ganzle MG, Lebeer S.: A taxonomic note on the genus Lactobacillus: Description of 23 novel genera, emended description of the genus Lactobacillus Beijerinck 1901, and union of Lactobacillaceae and Leuconostocaceae, Int J Syst Evol Microbiol, 2020 Apr; 70(4): 2782-2858, unless otherwise indicated. According to the reclassification, the bacteria of the family *Lactobacillaceae* are classified into the following 31 genera at present:
*Lactobacillus, Paralactobacillus*, *Holzapfelia*, *Amylolactobacillus*, *Bombilactobacillus*, *Companilactobacillus*, *Lapidilactobacillus*, *Agrilactobacillus*, *Schleiferilactobacillus*, *Loigolactobacillus*, *Lacticaseibacillus*, *Latilactobacillus*, *Dellaglioa*, *Liquorilactobacillus*, *Ligilactobacillus*, *Lactiplantibacillus*, *Furfurilactobacillus*, *Paucilactobacillus*, *Limosilactobacillus*, *Fructilactobacillus*, *Acetilactobacillus*, *Apilactobacillus*, *Levilactobacillus*, *Secundilactobacillus*, *Lentilactobacillus*, *Pediococcus*, *Convivina*, *Leuconostoc*, *Fructobacillus*, *Oenococcus*, and *Weissella.*

Among the lactic acid bacteria belonging to the family *Lactobacillaceae*, lactic acid bacteria belonging to the following 25 genera, including two genera existed from before the reclassification (*Lactobacillus* and *Paralactobacillus*) and 23 genera newly established by the reclassification, are used in preferred embodiments of the composition of the present invention;

*Lactobacillus*, *Paralactobacillus*, *Holzapfelia*, *Amylolactobacillus*, *Bombilactobacillus*, *Companilactobacillus*, *Lapidilactobacillus*, *Agrilactobacillus*, *Schleiferilactobacillus*, *Loigolactobacillus*, *Lacticaseibacillus*, *Latilactobacillus*, *Dellaglioa*, *Liquorilactobacillus*, *Ligilactobacillus*, *Lactiplantibacillus*, *Furfurilactobacillus*, *Paucilactobacillus*, *Limosilactobacillus*, *Fructilactobacillus*, *Acetilactobacillus*, *Apilactobacillus*, *Levilactobacillus*, *Secundilactobacillus*, and *Lentilactobacillus.*

In another preferred embodiment, among the lactic acid bacteria belonging to the family *Lactobacillaceae*, a lactic acid bacterium belonging to any of the genera *Lactiplantibacillus*, *Lactobacillus*, *Lacticaseibacillus*, *Companilactobacillus*, and *Limosilactobacillus* is used.

In another preferred embodiment, among the lactic acid bacteria belonging to the above genera, a lactic acid bacterium belonging to any of *Lactiplantibacillus plantarum, Lactobacillus acidophilus, Lactobacillus amylovorus*, *Lacticaseibacillus casei*, *Lacticaseibacillus paracasei*, *Companilactobacillus farciminis*, and *Limosilactobacillus mucosae* is used.

In another preferred embodiment, among the above lactic acid bacteria, a lactic acid bacterium belonging to any of *Lactiplantibacillus plantarum*, *Lactobacillus amylovorus*, *Lacticaseibacillus casei*, *Lacticaseibacillus paracasei*, *Companilactobacillus farciminis*, and *Limosilactobacillus mucosae* is used. In another preferred embodiment, among the above lactic acid bacteria, a lactic acid bacterium belonging to any of *Lactiplantibacillus plantarum*, *Lacticaseibacillus casei*, *Lacticaseibacillus paracasei*, *Companilactobacillus farciminis*, and *Limosilactobacillus mucosae* is used.

According to the classification before the reclassification, examples of lactic acid bacteria that can be used in preferred embodiments of the composition of the present invention include the followings:
*Lactobacillus delbrueckii*, *Lactobacillus delbrueckii* subsp. *bulgaricus*, *Lactobacillus delbrueckii* subsp. *lactis*, *Lactobacillus casei* (*Lacticaseibacillus casei* after the reclassification), *Lactobacillus paracasei* (*Lacticaseibacillus paracasei* after the reclassification), *Lactobacillus helveticus* (unchanged), *Lactobacillus acidophilus* (unchanged), *Lactobacillus crispatus* (unchanged), *Lactobacillus amylovorus* (unchanged), *Lactobacillus gallinarum* (unchanged), *Lactobacillus gasseri* (unchanged), *Lactobacillus paragasseri* (unchanged), *Lactobacillus oris* (*Limosilactobacillus oris* after the reclassification), *Lactobacillus rhamnosus (Lacticaseibacillus rhamnosus* after the reclassification), *Lactobacillus johnsonii* (unchanged), *Lactobacillus fermentum* (*Limosilactobacillus fermentum* after the reclassification), *Lactobacillus brevis* (*Levilactobacillus brevis* after the reclassification), *Lactobacillus plantarum* (*Lactiplantibacillus plantarum* after the reclassification), *Lactobacillus pentosus* (*Lactiplantibacillus pentosus* after the reclassification), *Lactobacillus paraplantarum* (*Lactiplantibacillus paraplantarum* after the reclassification), *Lactobacillus paracollinoides* (*Secundilactobacillus paracollinoides* after the reclassification), *Lactobacillus hammesii* (*Levilactobacillus hammesii* after the reclassification), *Lactobacillus farciminis* (*Companilactobacillus farciminis* after the reclassification), and *Lactobacillus mucosae* (*Limosilactobacillus mucosae* after the reclassification).

In a particularly preferred embodiment, *Lactiplantibacillus plantarum* (*Lactobacillus plantarum* before the reclassification) is used as the lactic acid bacterium. More specifically, *Lactiplantibacillus plantarum* (*Lactobacillus plantarum*) OLL2712 (hereinafter may be simply referred to as OLL2712) or a taxonomically identical strain thereof is used.

OLL2712 was deposited as an international deposition at the independent administrative agency, National Institute of Advanced Industrial Science and Technology (AIST), International Patent Organism Depositary (Tsukuba Central 6, 1-1-1 Higashi, Tsukuba, Ibaraki, Japan) on July 2, 2010, and given the accession number FERM BP-11262. As described in Budapest Notification No. 282 (http://www.wipo.int/treaties/en/notifications/budapest/treaty_budapest_282.html), the microorganism deposition service for patents of the independent administrative agency, National Institute of Advanced Industrial Science and Technology, International Patent Organism Depositary (IPOD, AIST) was succeeded to the independent administrative agency, National Institute of Technology and Evaluation, International Patent Organism Depositary (IPOD,NITE, Room 120, 2-5-8 Kazusakamatari, Kisarazu, Chiba, Japan), and therefore OLL2712 is now deposited at the independent administrative agency, National Institute of Technology and Evaluation, International Patent Organism Depositary (IPOD, NITE with the accession number FERM BP-11262 (depositor, Meiji Co., Ltd.).

In a particularly preferred embodiment, a strain substantially equivalent to OLL2712 can also be used as the lactic acid bacterium. Examples of substantially equivalent strains are as follows.
(1) Strains belonging to the genus *Lactiplantibacillus*, preferably *Lactiplantibacillus plantarum*, *Lactiplantibacillus pentosus*, or *Lactiplantibacillus paraplantarum*, more preferably *Lactiplantibacillus plantarum*, of which 16S rRNA gene sequence has a sequence identity of 90% or higher, preferably 95% or higher, more preferably 98% or higher, still more preferably 99% or higher, even more preferably 99.5% or higher, to the sequence of SEQ ID NO: 1.
(2) Strains having the same bacteriological properties as those of OLL2712.

A partial sequence (533 bp) of the 16S rRNA gene of OLL2712 is shown in the sequence listing as SEQ ID NO: 1.

The bacteriological properties of OLL2712 are listed below:
gram stain, positive; morphology, rod; lactic acid fermentation type, homolactic acid fermentation; aerobic growth, +; growth temperature, 15°C +, 45°C -; and
colony morphology: diameter, 1 to 2 mm; color, white; form, circular; elevation, convex (hemispherical); margin, entire; surface appearance, smooth; transparency, opaque; and consistency, butyrous (buttery).

In another particularly preferred embodiment, *Companilactobacillus farciminis* (*Lactobacillus farciminis* before the reclassification) OLL204082 is used as the lactic acid bacterium. More specifically, *Companilactobacillus farciminis* (*Lactobacillus farciminis*) OLL204082 (henceforth also simply referred to as OLL204082) or a strain taxonomically identical thereto is used.

OLL204082 was deposited as an international deposition at the independent administrative agency, National Institute of Technology and Evaluation, NITE Patent Microorganisms Depositary (NPMD, NITE, Room 122, 2-5-8 Kazusakamatari, Kisarazu, Chiba, Japan) on February 14, 2018, and given the accession number NITE BP-02635 (depositor, Meiji Co., Ltd.).

In another particularly preferred embodiment, a strain substantially equivalent to OLL204082 can also be used as the lactic acid bacterium. Examples of substantially equivalent strain are as follows.

(1) Strains belonging to the genus *Companilactobacillus*, preferably *Companilactobacillus farciminis*, *Companilactobacillus futsaii*, or *Companilactobacillus formosensis*, more preferably *Companilactobacillus farciminis*, of which 16S rRNA gene sequence has a sequence identity of 90% or higher, preferably 95% or higher, more preferably 98% or higher, still more preferably 99% or higher, even more preferably 99.5% or higher, to the sequence of SEQ ID NO: 2.
(2) Strains having the same bacteriological properties as those of OLL204082.

The bacteriological properties of OLL204082 are listed below:
cell morphology, rod; characteristics on culture medium (BL agar medium "Nissui", Nissui Pharmaceutical): circular, smooth, white, and convex; gram stain, positive; and catalase, negative.

The lactic acid bacterium contained in the composition of the present invention can be produced by culture. The culture conditions are not particularly limited so long as the desired effect is achieved.

In the composition of the present invention, the lactic acid bacterium may be contained in any state so long as it can exhibit the desired effect. For example, the lactic acid bacterium may be in the form of lactic acid bacterium cells themselves or culture of lactic acid bacterium (consisting of the lactic acid bacterium and culture supernatant). Cells of the lactic acid bacterium may be in a live state (live cells) or dead state (dead cells), so long as the desired effect can be achieved.

In one of preferred embodiments, the lactic acid bacterium is contained in the composition as dead bacterial cells. The dead bacterial cells can be obtained by sterilizing the lactic acid bacterium. The sterilization treatment is not particularly limited so long as the desired effect can be achieved, and can be performed by heating, or with sterilizing light (UV), ozone, chemicals, hyperosmotic pressure, or the like.

It is preferred that the dead bacterial cells are heat-treated dead bacterial cells obtained by heat-treating live bacterial cells. The heat treatment to obtain the heat-treated dead bacterial cells is not particularly limited so long as the desired effect is achieved, and is conducted at a temperature and time sufficient to kill the lactic acid bacterium used. Such conditions depend on the lactic acid bacterium used, but for example, 55°C or higher, preferably 60°C or higher, more preferably 65°C or higher, still more preferably 70°C or higher, and may be 80°C or higher, or 90°C or higher. The maximum heat treatment temperature may be appropriately determined, and it is, for example, 121°C or lower, and may be 100°C or lower, 90°C or lower, or 80°C or lower. Depending on the heat treatment temperature, the heat treatment time may be 1 minute or longer, 3 minutes or longer, 10 minutes or longer, 15 minutes or longer, 30 minutes or longer, or 45 minutes or longer. The longest heat treatment time can be, for example, 120 minutes or shorter, 100 minutes or shorter, 90 minutes or shorter, or 80 minutes or shorter.

The lactic acid bacterium cells to be contained in the composition can be prepared as a dried product, suspension, paste, gel, or the like, irrespective of whether they are live or dead cells.

### [Use]

The composition of the present invention can be used for inducing autophagy. The composition of the present invention can be used particularly for inducing autophagy in the intestine, more specifically for inducing autophagy in intestinal epithelial cells and intestinal epithelial stem cells . Intestinal epithelial cells include absorptive epithelial cells, Paneth cells, and goblet cells. Intestinal epithelial cells and intestinal epithelial stem cells are preferably cells that are not infected by pathogens (non-infected cells), cells that are not cancerized (non-cancerous cells), or non-infected and non-cancerous cells. For the present invention, the term induction of autophagy refers to induction of autophagy not accompanied by cell death, unless otherwise stated. Induction of autophagy referred to for the present invention and the embodiment thereof does not include induction of autophagy accompanied by cell death, as reported in Non-patent document 15 mentioned above.

Whether a composition or ingredient activates autophagy or not can be evaluated by, for example, a DALGreen assay using appropriate cells. DALGreen is a fluorescent dye that can detect autophagy, which is a degradation process for recycling or metabolizing unnecessary or discarded proteins and other substances in cells. When DALGreen is added to cultured cells, it penetrates live plasma membranes and is incorporated into autophagosomes formed by autophagy induction. Subsequently, DALGreen fluorescence increases in autolysosomes that have fused with lysosomes to form an acidic environment. By adding an appropriate amount of the composition or ingredient to be evaluated to such a system, incubating them for several hours to several days in an environment suitable for cell culture, then confirming and measuring fluorescence intensity by such a method as fluorescence microscopy, whether or not autophagy has been activated by the added composition or ingredient can be evaluated. Whether a certain composition or ingredient activates autophagy without cell death can be evaluated according to whether it is found to activate autophagy by the method described above and whether cell viability is decreased or not in a cell proliferation assay (e.g., WST-1 assay described in the section of Examples).

In a preferred embodiment, the composition can be used for maintenance of homeostasis or maintenance of metabolism in the intestine, especially in intestinal epithelial cells and intestinal epithelial stem cells, more specifically in any cells selected from the group consisting of absorptive epithelial cells, Paneth cells, goblet cells, and intestinal epithelial stem cells; for maintenance of number, maintenance of function or regeneration of any cells selected from the group consisting of absorptive epithelial cells, Paneth cells, goblet cells, and intestinal epithelial stem cells; for keeping the intestinal tract healthy and maintenance of intestinal homeostasis; and for maintenance of intestinal barrier function in healthy individuals, and preventing inflammation in the intestinal tract of healthy individuals.

The uses of the composition of the present invention may exclude any use selected from the group consisting of:
improvement of intestinal inflammation induced by lipopolysaccharides (LPS), suppression of increase in intestinal permeability caused by LPS, prevention of intestinal barrier dysfunction caused by LPS;
suppression of intestinal epithelial barrier dysfunction, restoration of the barrier function, protection against increase in mucosal permeability associated with sodium dextran sulfate-induced colitis, treatment of gastrointestinal diseases such as inflammatory bowel disease;
prevention and treatment of alcohol-induced tissue injury (disruption of tight junctions in the colon, intestinal mucosal barrier dysfunction, liver damage), especially alcoholic hepatitis;
promotion of autophagy via expression of membrane ATG 16L 1 protein, and suppression of inflammatory IL-1β expression in *Salmonella-infected* intestinal epithelial cells (IECs), treatment of infectious and autoimmune diseases caused thereby;
treatment of gluten-specific enteropathy, and restoration from abnormality in certain toxic parameters induced by gluten in enteritis.

### [Composition]

### (Food composition, etc.)

The composition of the present invention can be in the form of a food composition or pharmaceutical composition. Food and drug are not limited to those for humans, and may be those for animals other than human, unless especially indicated. The food may be a common food, functional food, or nutritional composition, or a therapeutic diet (diet for the purpose of treatment, for which a medical practitioner writes a dietary prescription, and which is cooked by a dietitian or the like according to the prescription), dietetic food, ingredient-modified food, care food, or treatment-supporting food, unless especially indicated. The food is not limited to a solid food, but it may be a food in the form of liquid, for example, drink, drinkable preparation, liquid food, or soup, unless especially indicated. Functional food refers to a food that can give a predetermined functionality to a living body, and includes health foods at large, such as foods for specified health uses (abbreviated as "Tokuho" in Japanese, including conditional foods for specified health use), foods with function claims, foods with health claims including foods with nutrient function claims, foods for special dietary uses, supplements (for example, those of various kinds of dosage forms such as tablet, coated tablet, sugar-coated tablet, capsule and solution), and cosmetic food (for example, diet foods). In the present invention, the "functional foods" include health foods to which the health claim based on the food standards of CODEX (JOINT FAO/WHO FOOD STANDARDS PROGRAMME CODEX ALIMENTARIUS COMMISSION) is applied.

### (Subject)

The composition of the present invention is suitable to be ingested by or administered to subjects for whom it is preferable to activate autophagy, especially to activate autophagy in the intestine, more specifically to activate autophagy in intestinal epithelial cells and intestinal epithelial stem cells. The composition of the present invention is also suitable to be ingested by or administered to subjects for whom it is desirable to maintain a healthy intestinal tract; subjects for whom it is desirable to maintain intestinal homeostasis; subjects for whom it is desirable to maintain a healthy intestinal barrier function; or subjects for whom it is desirable to reduce the risk of inflammation in the intestinal tract. For any type of subjects, it is preferred that they are healthy (e.g., with normal intestinal barrier function, no inflammation in the intestinal tract, no disease in the intestinal tract (no infection and/or no cancer)).

The composition of the present invention is also suitable to be used for inducing autophagy in healthy subjects, especially for inducing autophagy in the intestines of healthy subjects, more specifically for inducing autophagy in intestinal epithelial cells and intestinal epithelial stem cells of healthy subjects. The composition of the present invention can be used non-therapeutically. Non-therapeutic means to be not intended to treat diseases.

For the present invention, the term "healthy" means to have no infectious disease, no cancer, or no infectious disease and no cancer, unless otherwise noted. The absence of infectious disease, cancer, or infectious disease and cancer can be determined by physicians. The infectious disease is defined as a condition of having such symptoms as pain, inflammation, and fever due to infection by a pathogen.

The subjects mentioned above include adults (15 years old or older), middle-aged and older persons, elderly persons (65 years old or older), persons in or after sickness, pregnant women, childbearing women, infants, and children. In addition, the composition of the present invention is particularly suitable for subjects for whom it is desirable to activate autophagy in their daily lives, because the active ingredient is a lactic acid bacterium having been used in a food from very old years, and therefore it is suitable for ingestion for a long period of time.

### (Administration route)

Although the composition of the present invention may be administered parenterally, for example, by tube administration (by gastric fistula, cecal fistula, or the like), or may be administered nasally or orally, it is preferably orally administered.

### (Content and dose of active ingredient)

The content of the lactic acid bacterium in the composition of the present invention may be such an amount that the desired effect is exhibited. The content of the lactic acid bacterium in the composition may be appropriately determined in consideration of various factors such as age, weight, and conditions of the subject who will ingest or will be administered the composition. One unit of the composition may consist of an amount to be ingested or administered per day or per one time (it may be administered or ingested once a day or multiple times a day, e.g., three times a day). The content of the lactic acid bacterium per one unit of the composition can be, for example, 1 × 10⁷ cells or more, or may be 1 × 10⁸ cells or more, 5 × 10⁸ cells or more, 1 × 10⁹ cells or more, or 5 × 10⁹ cells or more, and it is preferably 1 × 10¹⁰ cells or more, more preferably 1 × 10¹¹ cells or more, still more preferably 2 × 10¹¹ cells or more. No matter how the minimum amount is defined, the maximum amount may be appropriately determined, and it may be, for example, 1 × 10¹⁴ cells or less, 1 × 10¹³ cells or less, or 1 × 10¹² cells or less.

In any case where the content of the lactic acid bacterium is as described above, one unit can be, for example, 0.5 g or heavier, 1 g or heavier, 5 g or heavier, 10 g or heavier, 20 g or heavier, or 30 g or heavier. When the composition is in the form of a food such as fermented milk, one unit can be an amount that is easily consumed in a single serving as a food, for example, 50 g or more, 60 g or more, 70 g or more, 80 g or more, 90 g or more, or 100 g or more. No matter how the minimum amount is defined, the maximum amount may be appropriately determined, and it can be 500 g or less, 400 g or less, 300 g or less, 200 g or less, 150 g or less, or 125 g or less.

The composition comprises lactic acid bacteria that have been used in a food from very early years as the active ingredient. Therefore, the composition may be taken repeatedly or over a long period of time, and it can be ingested or administered continuously for, for example, 3 days or longer, preferably 1 week or longer, more preferably 4 weeks or longer, especially preferably 1 month or longer.

### (Other ingredients and additives)

The composition of the present invention may comprise another active ingredient or nutritional ingredient acceptable for foods or drugs. Examples of such an ingredient include amino acids (for example, lysine, arginine, glycine, alanine, glutamic acid, leucine, isoleucine, and valine), saccharides (glucose, sucrose, fructose, maltose, trehalose, erythritol, maltitol, paratinose, xylitol, and dextrin), electrolytes (for example, sodium, potassium, calcium, magnesium salts etc.), vitamins (for example, vitamin A, vitamin B1, vitamin B2, vitamin B6, vitamin B12, vitamin C, vitamin D, vitamin E, vitamin K, biotin, folic acid, pantothenic acid, and nicotinic acid), minerals (for example, copper, zinc, iron, cobalt, and manganese), antibiotics, dietary fibers, proteins, lipids, and so forth.

The composition may also further comprise an additive acceptable for foods or drugs. Examples of such an additive include inactive carriers (solid and liquid carriers), excipients, surfactants, binders, disintegrating agents, lubricants, dissolving aids, suspending agents, coating agents, colorants, preservatives, buffering agents, pH adjustors, emulsifiers, stabilizers, sweeteners, antioxidants, perfumes, acidulants, and natural substances. More specific examples include water, other aqueous solvents, pharmaceutically acceptable organic solvents, collagen, polyvinyl alcohol, polyvinylpyrrolidone, carboxyvinyl polymer, sodium alginate, water-soluble dextran, water-soluble dextrin, carboxymethyl starch sodium, pectin, xanthan gum, gum arabic, casein, gelatin, agar, glycerin, propylene glycol, polyethylene glycol, vaseline, paraffin, stearyl alcohol, stearic acid, human serum albumin, mannitol, sorbitol, lactose, sucralose, stevia, aspartame, acesulfame potassium, citric acid, lactic acid, malic acid, tartaric acid, phosphoric acid, acetic acid, fruit juice, vegetable juice, and so forth.

### (Dosage form or shape)

The composition of the present invention in the form of a food may be prepared in an arbitrary form such as solid, liquid, mixture, suspension, powder, granule, paste, jelly, gel, and capsule. The composition of the present invention in the form of a food can be made in such an arbitrary form as dairy product, supplement, confectionery, drink, drinkable preparation, seasoning, processed food, daily dish, and soup. More specifically, the composition of the present invention may be in the form of fermented milk, lactic acid bacterium beverage, lactic beverage, milk beverage, soft drink, ice cream, tablet, cheese, bread, biscuit, cracker, pizza crust, special formula powdered milk, liquid food, food for sick persons, nutritional food, frozen food, processed food, or the like, and can also be in the form of granule, powder, paste, thick solution, etc. to be mixed with beverages and foods for ingestion. Fermented milk means fermented milk and lactic acid beverages as defined in the "Ministerial Ordinance on Milk and Milk products Concerning Compositional Standards etc." issued by Ministry of Health and Welfare, Japan (hereinafter referred to as the "Ministerial Ordinance on Milk, etc."). Fermented milk defined in the Ministerial Ordinance on Milk, etc. is a product prepared by fermenting milk or milk product having a nonfat milk solid content not lower than that of milk with a lactic acid bacterium or yeast and making it into paste or liquid, or a product obtained by freezing the foregoing.

The composition of the present invention as a pharmaceutical can be made into any dosage form suitable for oral administration, including solid dosage forms such as tablet, granule, powder, pill, and capsule, liquid dosage forms such as solution, suspension, and syrup, gel, aerosol, and so forth.

### (Others)

In the manufacture of the composition of the present invention, timing of adding the lactic acid bacterium can be appropriately chosen. Unless the characteristics of the lactic acid bacterium are not markedly degraded, the timing of the addition is not particularly limited. For example, a composition in the form of 100 mL of fruit juice-flavored soft drink can be prepared by mixing the raw materials (acidifier, sweetener, stabilizer, fruit juice, flavor, lactic acid bacterium cells, and water) and heat-treated OLL 2712 (about 10⁹ cells suspended in ion exchanged water and heat-treated at 95°C for 3 minutes) and bottling the mixture.

The composition of the present invention can have an indication describing the purpose of use (intended use) and can also have an indication describing a recommendation for ingestion thereof by specific subjects. The indication may be a direct or indirect indication. Examples of the direct indication include descriptions on tangible articles such as the product itself, package, container, label, and tag, and examples of the indirect indication includes advertising and campaign activities using such places or means as web site, shop, pamphlet, exhibition, seminar such as media seminar, book, newspaper, magazine, television, radio, postal matter, E-mail, and sound.

Examples of the use and function to be indicated on the composition as a food include "for inducing autophagy", "for inducing autophagy in the intestines", "for maintenance of homeostasis", "for maintenance of metabolism", "for keeping the intestinal tract healthy", "for maintaining the intestinal barrier function in healthy people", "for maintaining homeostasis decreasing with age", "for maintaining metabolism decreasing with age", and so forth.

Hereafter, the present invention will be more specifically explained with reference to examples.

### Examples

### [Example 1]

*Lactobacillus plantarum* (*Lactiplantibacillus plantarum*) OLL2712 (accession number FERM BP-11262) and other lactic acid bacteria belonging to the family *Lactobacillaceae*, 10 strains in total (*Lactobacillus plantarum* (4 strains), *Lactobacillus acidophilus, Lactobacillus casei* (*Lacticaseibacillus casei*), *Lactobacillus farciminis* (*Companilactobacillus farciminis*), *Lactobacillus paracasei* (*Lacticaseibacillus paracasei), Lactobacillus mucosae* (*Limosilactobacillus mucosae),* and *Lactobacillus amylovorus*, see table mentioned below), were cultured in the MRS medium for 18 hours, centrifuged to remove the culture supernatant, suspended in PBS, centrifuged again, and washed twice. The cells were suspended in distilled water and washed twice in the same manner. The cells were suspended in 1 mL of distilled water and lyophilized. The lyophilized bacterial cells were suspended in distilled water and heated at 75°C for 60 minutes. The bacterium in an amount of 100 µg/mL diluted in the medium (equivalent to about 3 × 10⁸ cells/mL in the case of OLL2712) was added to human colon cancer-derived Caco-2 cells for autophagy activity evaluation (DALGreen assay).

In the assay, there were used cells obtained with only the medium (Cont. (no stimulation)) as a negative control, and cells obtained with addition of raloxifene diluted in the medium as a positive control. For each well, 5 fields of view were taken, and values obtained by dividing luminances (integrated) of luminescence of DALGreen by the number of cells for 2 wells each for the samples, 10 fields of view in total, as the luminescence intensities were compared with the value of Cont., of which activity was taken as 1.

**[Table 1]**

| Bacterial strain used | |
|---|---|
| Bacterial species | Accession number, etc. |
| *L. plantarum* | JCM1149^{T} |
| | FERM BP-11262 |
| | P2100301 |
| | P2100302 |
| *L. acidophilus* | JCM1132^{T} |
| *L. casei* | JCM1134^{T} |
| *L. farciminis* | JCM1097^{T} |
| *L. paracasei* | JCM8130^{T} |
| *L. mucosae* | JCM12515^{T} |
| *L. amylovorus* | JCM1126^{T} |

The strains of which numbers mentioned in the column of Accession number etc. begin with JCM can be obtained from the independent administrative agency, RIKEN BioResource Research Center, Japan Collection of Microorganisms (RIKEN BRC-JCM, Japan) by using the accession numbers, JCM1149^{T}, JCM1132^{T}, JCM1134^{T}, JCM1097^{T}, JCM8130^{T}, JCM12515^{T}, and JCM1126^{T}. Those of which numbers begin with P21 are stored by Meiji Co., Ltd., Meiji Innovation Center (1-29-1 Nanakuni, Hachioji, Tokyo, Japan, 192-0919).

As a result, autophagy-inducing effects higher than that of Cont. were observed for at least one of the cells obtained by 5 and 24 hours incubations (Fig. 1). When the lower end of the error bar of each sample was higher than the upper end of the error bar of Cont., the result was considered to indicate the presence of autophagy-inducing effect. In Fig. 1, * indicates p<0.05, ** indicates p<0.01 vs Cont., and the numbers indicated over the columns are p-values.

The LDH (lactate dehydrogenase) assay was also performed, and some LDH activity was observed for *L. acidophilus* and *L. amylovorus.* However, the both strains showed no decrease in viability in the cell growth assay (WST-1 assay). Therefore, no cytotoxicity was observed for the both strains (Fig. 2).

### [Example 2]

For the bacterial species of which standard strains showed autophagy activity in Example 1 (*Lactobacillus acidophilus*, *Lactobacillus casei* (*Lacticaseibacillus casei*), *Lactobacillus farciminis* (*Companilactobacillus farciminis*), *Lactobacillus paracasei* (*Lacticaseibacillus paracasei*), *Lactobacillus mucosae* (*Limosilactobacillus mucosae*), and *Lactobacillus amylovorus*, see the table mentioned below), autophagy activity evaluation (DALGreen assay) was performed with increased numbers of strains.

**[Table 2]**

| Bacterial strain used | |
|---|---|
| Bacterial species | Accession number, etc. |
| *L. acidophilus* | JCM1023 |
| *L. casei* | P2100303 |
| | P2100304 |
| *L. farciminis* | NITE BP-02635 |
| *L. paracasei* | JCM1053 |
| *L. mucosae* | P2100305 |
| *L. amylovorus* | P2100306 |

The strains of which numbers mentioned in the column of Accession number etc. begin with JCM can be obtained from the independent administrative agency, RIKEN BioResource Research Center (RIKEN BRC), Japan Collection of Microorganisms (RIKEN BRC-JCM, Japan), and those of which numbers begin with P21 are stored by Meiji Co., Ltd., Meiji Innovation Center (1-29-1 Nanakuni, Hachioji, Tokyo, Japan, 192-0919).

As a result, higher autophagy-inducing effects compared with that of Cont. were observed for all the species and strains of bacteria (Fig. 3). When lower end of the error bar was higher than the upper end of the error bar of Cont., it was determined that the strain has an autophagy-inducing effect.

### [Reference Example]

Evaluation of autophagy activity (DALGreen assay) was performed for a microorganism that is not a lactic acid bacterium belonging to the family *Lactobacillaceae*, *Streptococcus thermophilus* 1131 strain (this strain can be isolated from Meiji Bulgaria Yogurt LB81 Plain (Meiji Co., Ltd.)), in the same manner as those of Examples 1 and 2. As a result, no autophagy-inducing effect was observed for that strain (Fig. 4).

### [Details of the experimental methods used in the examples and reference examples]

### (DALGreen assay method)

(1) Seed cells on a 96-well plate (MS-8096F, Sumitomo Bakelite) at a density of 1 × 10⁴ cells/100 µL/well, and culture them overnight (preferably 24 hours) at 37°C.
(2) Dilute DALGreen with the medium to a final concentration of 1 µM.
(3) Remove the medium used in (1) and add 50 µL/well of the solution prepared in (2).
(4) Incubate the plate in a CO₂ incubator at 37°C for 30 minutes.
(5) Remove the medium used in (4) and wash the cells with 50 µL of HBSS(+).
(6) Add 50 µL of a sample diluted with the medium to each well and incubate the plate for 5 hours or 24 hours at 37°C in a CO₂ incubator.
(7) Wash the cells with 50 µL of HBSS(+) after 5 or 24 hours.
(8) Dilute Hoechst 33342 500-fold (2 µg/mL) with the medium having the following composition based on Dulbecco's Modified Eagle Medium (DMEM, Sigma #D1145), and add 50 µL of the solution to each well.

**[Table 3]**

| Phenol red-free Caco-2 medium | | |
|---|---|---|
| | Final concentration | Sources, etc. |
| DMEM | | Sigma, D1145 |
| Fetal bovine serum | 10 % | Biowest, S1810-500 |
| Penicillin-streptomycin (100x) | 1 % | Gibco, 15140-122 |
| Non-essential amino acids (100x) | 1 % | Sigma, M7145 |
| L-Glutamine | 4 mM | Thermo, 25030-081 |
| Sodium pyruvate | 1 mM | Wako, 190-14881 |

Addition of L-glutamine and sodium pyruvate gives almost the same composition as that of Sigma #D6429.
(9) Incubate the plate in a CO₂ incubator at 37°C for 15 minutes.
(10) Observe the cells with a fluorescence microscope.

### (WST-1 assay method)

Takara's Premix WST-1 Cell Proliferation Assay System (#MK400) is used as the kit.
(1) Seed cells on a 96-well plate at a density of 1 × 10⁴ cells/100 µL/well.
(2) Stimulate the cells with a sample on the next day.
(3) Incubate the cells in a CO₂ incubator at 37°C for an arbitrary period of time such as 5 and 24 hours, and then add Premix WST-1 (Takara) in a volume of 10 µL/well.
(4) Incubate the cells in a CO₂ incubator at 37°C for an arbitrary period of time.
(5) Measure absorbance at 440 nm (at 600 nm or longer for the control) after 15 minutes, 30 minutes, 1 hour, and 2 hours (adopt times that give an absorbance not higher than 1.0).

### (LDH assay method)

Cytotoxicity LDH Assay Kit-WST (#CK12) of Dojin Chemical is used as the kit.
(1) Seed cells at a density of 1 × 10⁴ cells/100 µL/well.
(2) Add a sample in a volume of 50 µL/well on the next day.
(3) Incubate the plate in a CO₂ incubator at 37°C for an arbitrary period of time such as 5 and 24 hours, and before adding lysis buffer, add 50 µL of the medium to make the liquid volume match that defined in the protocol.
(4) Add 10 µl/well of the lysis buffer to the well of High Cont.
(5) Incubate the plate in a CO₂ incubator at 37°C for 30 minutes.
(6) In the case of culture supernatant assay, centrifuge the culture at this stage (250 x g, 2 minutes) to collect 100 µL of supernatant.
(7) Add 100 µl/well of the working buffer to all wells.
(8) Allow reactions at RT for 10 minutes with light shielding.
(9) Add 50 µl/well of the stop solution.
(10) Measure absorbance at 490 nm.

### [Preparation Example 1: Fermented milk]

To a mixture comprising raw milk, skimmed milk powder, cream, sugar, and stevia, *Lactobacillus delbrueckii* subsp. *bulgaricus* and *Streptococcus thermophilus* were added as the lactic acid bacterium starter, and fermentation was allowed at 43°C for about 3 hours to prepare yogurt. Heat-treated *Lactobacillus plantarum* OLL2712 (accession number, FERM BP-11262) cells were added to the prepared yogurt to produce fermented milk comprising *Lactobacillus plantarum* OLL2712 strain cells. The amount of the heat-treated dead bacterial cells in the fermented milk was 5 × 10⁹ or more per 112 g. The heat treatment of the lactic acid bacterium was performed by concentrating the bacterial cells to the above density and then heating them at 60°C for 10 minutes.

Fermented milk can be produced in the same manner by using the other bacterial species or strains listed in Table 1 or 2 instead of the *Lactobacillus plantarum* OLL2712 strain.

### [Preparation Example 2: Soft drink]

Raw materials (acidifier, sweetener, stabilizer, fruit juice, flavor, lactic acid bacterium cells (heat-treated *Lactobacillus plantarum* OLL2712 strain (accession number, FERM BP-11262) cells), and water) were mixed to obtain a soft drink, and it was bottled in a volume of about 100 mL. The amount of the lactic acid bacterium in the soft drink was about 10¹⁰ cells/100 mL. The heat treatment of the lactic acid bacterium was performed by concentrating the bacterial cells to the above density, suspending them in ion-exchanged water, and heating the suspension at 95°C for 3 minutes.

Soft drinks can be produced in the same manner by using the other bacterial species or strains listed in Table 1 or 2 instead of the *Lactobacillus plantarum* OLL2712 strain.

### Free Text of Sequence Listing

SEQ ID NO: 1, Partial sequence of 16S rRNA gene of OLL2712 (FERM BP-11262)
SEQ ID NO: 2, Partial sequence of 16S rRNA gene of OLL204082 (NITE BP-02635)

## Claims

1. A composition for inducing autophagy, the composition comprising bacterial cells of a lactic acid bacterium belonging to the family *Lactobacillaceae.*

2. The composition according to claim 1, wherein the lactic acid bacterium is a lactic acid bacterium belonging to any of the genera *Lactiplantibacillus*, *Lactobacillus*, *Lacticaseibacillus*, *Companilactobacillus*, and *Limosilactobacillus.*

3. The composition according to claim 1, wherein the lactic acid bacterium is a lactic acid bacterium belonging to any of *Lactiplantibacillus plantarum*, *Lactobacillus acidophilus*, *Lactobacillus amylovorus*, *Lacticaseibacillus casei*, *Lacticaseibacillus paracasei*, *Companilactobacillus farciminis*, and *Limosilactobacillus mucosae.*

4. The composition according to any one of claims 1 to 3, which is for inducing autophagy in the intestine.

5. The composition according to any one of claims 1 to 3, which comprises 1 × 10⁹ or more of bacterial cells of the lactic acid bacterium per one unit.

6. The composition according to any one of claims 1 to 3, which comprises 2 × 10¹¹ or more of bacterial cells of the lactic acid bacterium per one unit.

7. The composition according to any one of claims 1 to 3, wherein the bacterial cells of the lactic acid bacterium are dead bacterial cells.

8. The composition according to 7, wherein the dead bacterial cells are heat-treated dead bacterial cells.
